# FASCICULE DE BREVET EUROPEEN

(11) **EP 1 340 485 B1**
(45) Date de publication et mention de la délivrance du brevet: **13.02.2008**
(21) Numéro de dépôt: 03290446.8
(22) Date de dépôt: 25.02.2003
(51) Int. Cl.: A61K 8/04, A61K 8/73, A61K 8/81, A61Q 5/02, A61Q 19/10

(54) **Mousse aérosol comprenant l'association d'un polymère cationique de vinylpyrrolidone et d'un composé cellulosique non ionique et utilisations en cosmétique**
Aerosolschaumzusammensetzung enthaltend ein kationisches Vinylpyrrolidon-Polymer und eine nicht-ionische cellulosehaltige Verbindung und deren kosmetische Verwendung
Aerosol foam composition containing a combination of a cationic vinylpyrrolidone polymer and a nonionic cellulosic compound and its use in cosmetics

(30) Priorité: 01.03.2002 FR 0202648
(43) Date de publication de la demande: 03.09.2003
(73) Titulaire: L'ORÉAL, 75008 Paris (FR)
(72) Inventeur: Ribery, Delphine, 92300 Levallois Perret (FR); Penverne, Isabelle, 92400 Courbevoie (FR)
(74) Mandataire: Miszputen, Laurent

(56) Documents cités:
- EP-A- 1 097 701
- EP-A- 1 169 998
- WO-A-99/66888
- DE-C- 4 343 378
- GB-A- 2 270 259
- US-A- 4 761 273
- US-A- 5 900 229
- US-A- 6 063 368
- US-A1- 2001 022 967
- DATABASE CA [en ligne] CHEMICAL ABSTRACTS SERVICE, COLUMBUS, OHIO, US; ANON.: "Hair mousse composition" retrieved from STN Database accession no. 125:338679 XP002227673 & RESEARCH DISCLOSURE (1996), 390, P 660, NO. 39029,

## Description

L' invention concerne une composition moussante comprenant l'association d'un polymère cationique de vinylpyrrolidone et d'un composé cellulosique, le dispositif contenant cette composition ainsi que l'utilisation de cette composition en cosmétique ou dermatologie notamment pour le nettoyage des matières kératiniques telles que la peau, les cheveux ou le cuir chevelu.

Il existe actuellement sur le marché de l'hygiène corporelle des gels lavants automoussants conditionnés dans des dispositifs aérosols à 2 compartiments. On connaît également des produits pour la douche conditionnés dans un dispositif aérosol comportant un seul compartiment.

Un des souhaits des consommateurs est de disposer de produits apportant dans un même geste, nettoyage et soin de la peau, tout en profitant du plaisir apporté par une mousse abondante qui se rince facilement. La présentation des produits de nettoyage en aérosol permet, grâce au propulseur de générer rapidement, par action sur un bouton poussoir, une mousse abondante que l'on étale ensuite sur le corps.

La rigidité de la mousse générée à la sortie de l'aérosol est une caractéristique importante. En effet, c'est elle qui va procurer au consommateur, lors de la prise en main et de l'étalement, une sensation de densité et d'effet soin.

La demanderesse a découvert de manière surprenante qu'en utilisant une composition associant un polymère cationique de vinylpyrrolidone et un composé cellulosique dans une base lavante constituée d'un tensio-actif détergent, on obtenait un effet de synergie au niveau de la rigidité de la mousse. Cela procure ainsi à l'utilisateur une sensation de densité lors de la prise en main et lors de l'étalement, renforçant ainsi la notion de soin.

Cette découverte est à la base de la présente invention qui a pour objet une composition moussante comprenant :
(a) au moins un agent propulseur ;
(b) au moins une phase liquide contenant dans un milieu aqueux cosmétiquement acceptable :
   (i) un mélange de tensio-actifs détergents constitué d'au moins un agent tensioactif anionique et d'au moins un agent tensioactif amphotère;
   (ii) au moins un polymère cationique de vinylpyrrolidone choisi parmi les copolymères vinylpyrrolidone méthacrylate de diméthylaminoéthyle quaternisés par du sulfate de diéthyle et
   (iii) au moins un éther de cellulose non ionique choisi parmi les alkylcelluloses ; les hydroxyalkylcelluloses ; les celluloses mixtes hydroxyalkyl-alkylcelluloses.

L'invention a aussi pour objet un dispositif aérosol comprenant dans un même compartiment la composition moussante.

Un autre objet de l'invention consiste en l'utilisation de ladite composition pour la préparation d'un produit cosmétique ou dermatologique et notamment d'un produit de nettoyage des matières kératiniques.

Un autre objet de l'invention consiste en un procédé de nettoyage des matières kératiniques consistant à produire une mousse à la sortie d'un dispositif aérosol dans lequel est conditionnée la composition de l'invention et à l'appliquer sur lesdites matières kératiniques puis après un éventuel temps de pose, à procéder au rinçage.

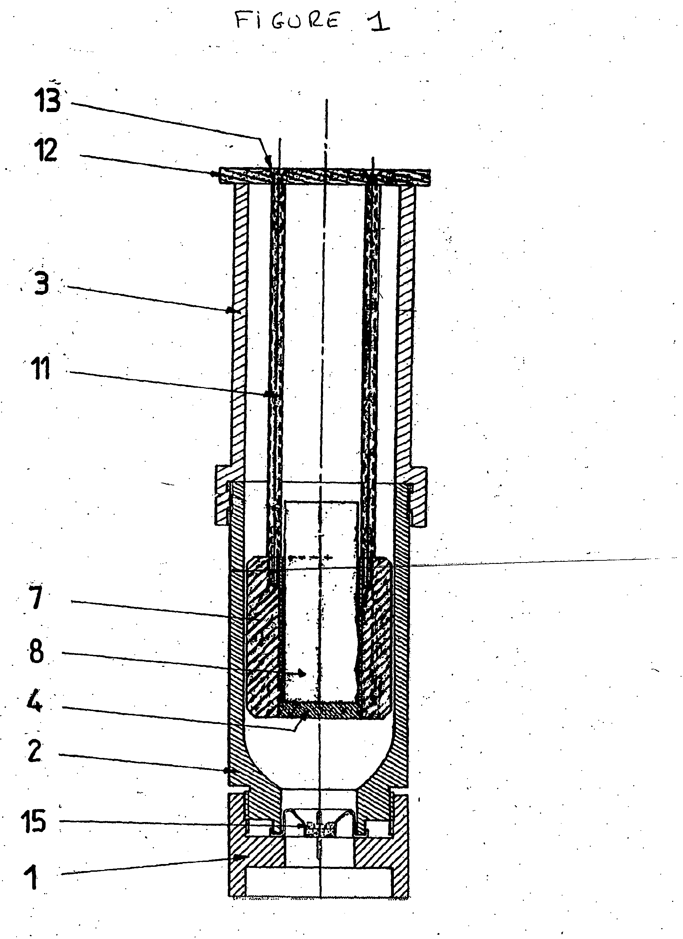

La figure 1 représente un dispositif permettant la mesure de la rigidité d'une mousse.

Les matières kératiniques au sens de la présente invention comprennent la peau (corps et visage), le cuir chevelu et les cheveux.

Les polymères cationiques de vinylpyrrolidone (PVP) utilisables conformément à l'invention sont choisis plus particulièrement parmi les copolymères vinylpyrrolidone / méthacrylate de diméthylaminoéthyle quaternisés par du sulfate de diéthyle, vendus sous les dénominations GAFQUAT 734, 755, 755 S et 755 L par la société I.S.P.

On utilisera plus particulièrement le Polyquaternium-11.

La concentration en ces polymères cationiques de vinylpyrrolidone varie de préférence de 0,01 à 5 % et encore plus préférentiellement de 0,05 à 2 % en poids environ de ce poids.

Par composé cellulosique, on entend selon l'invention tout composé polysaccharidique possédant dans sa structure des enchaînements de résidus glucose unis par des liaisons β-1,4. Outre les celluloses non substituées, les dérivés de celluloses peuvent être anioniques, cationiques, amphotères ou non-ioniques. Parmi ces dérivés, on distingue les éthers de celluloses, les esters de celluloses et les esters éthers de celluloses. Parmi les esters de celluloses, on trouve les esters inorganiques de cellulose (nitrates, sulfates ou phosphates de cellulose...), les esters organiques de cellulose (monoacétates, triacétates, amidopropionates, acétatebutyrates, acétatepropionates ou acétatetrimellitates de cellulose....) et les esters mixtes organique/inorganique de cellulose tels que les acétatebutyratesulfates et les acétatepropionatesulfates de cellulose. Parmi les esters éthers de cellulose, on peut citer les phtalates d'hydroxypropylméthylcellulose et les sulftates d'éthylcellulose.

Les composés cellulosiques de l'invention sont choisis parmi les éthers de cellulose anioniques, cationiques ou non-ioniques.

Parmi les éthers de cellulose non ioniques, on utilisera peut citer les alkylcelluloses telles que les méthylcelluloses et les éthylcelluloses ; les hydroxyalkylcelluloses telles que les hydroxyméthylcelluloses, les hydroxyéthylcelluloses et les hydroxypropylcelluloses ; les celluloses mixtes hydroxyalkyl-alkylcelluloses telles que les hydroxypropylméthylcelluloses, les hydroxyéthyl-méthylcelluloses, les hydroxyéthyl-éthylcelluloses et les hydroxybutyl-méthylcelluloses.

On utilisera plus particulièrement une hydroxypropyl-méthylcellulose.

Le ou les composés cellulosiques conformes à l'invention représentent de préférence de 0,01 à 5 % et encore plus préférentiellement de 0,05 à 2 % en poids par rapport au poids total de la composition.

On peut conditionner les compositions de nettoyage de la présente invention dans des dispositifs aérosols en présence de n'importe quel agent propulseur usuellement employé pour la préparation de compositions aérosols. On peut citer en particulier les gaz hydrocarbonés, comme par exemple le propane, le n-butane, l'isobutane et leurs mélanges; les gaz fluorés comme par exemple le chlorodifluorométhane, le dichlorodifluorométhane, le difluoroéthane, le chlorodifluoroéthane, le dichlorotétrafluoroéthane, etc. et leurs mélanges ; les gaz hydrofluorocarbonés ; le dimethyl ether et les mélanges de dimethyl ether avec un ou plusieurs gaz hydrocarbonés ; l'azote, l'air et le dioxyde de carbone et leurs mélanges peuvent également être utilisés comme gaz propulseurs dans la présente invention. De façon préférentielle, on utilise dans la présente invention des gaz hydrocarbonés ayant de 2 à 6 atomes de carbone et plus particulièrement un mélange iso-butane, propane, n-butane. Le ou les gaz propulseurs sont présents dans le dispositif à raison de 0,1 à 15% en poids et plus préférentiellement de 1 à 8% en poids par rapport au poids total de la composition.

Les tensioactifs détergents utilisables dans la présente invention sont des mélanges de tensio-actifs détergents constitué d'au moins un agent tensioactif anionique et d'au moins un agent tensioactif amphotère.

Comme tensioactifs anioniques utilisables dans la présente invention, on peut notamment mentionner les sels, en particulier les sels de métaux alcalins tels que les sels de sodium, les sels d'ammonium, les sels d'amines, les sels d'aminoalcools ou les sels de métaux alcalino-terreux, par exemple, de magnésium, des types suivants : les alkylsulfates, les alkyléthersulfates, les alkyl-amidoéthersulfates, les alkylarylpolyéthersulfates, les monoglycéride-sulfates ; les alkylsulfonates, les alkylamidesulfonates, les alkylarylsulfonates, les α-oléfine-sulfonates, les paraffine-sulfonates ; les alkylsulfosuccinates, les alkyléthersulfosuccinates, les alkylamide-sulfosuccinates ; les alkylsulfoacétates ; les acylsarcosinates ; et les acylglutamates, les groupes alkyle et acyle de tous ces composés comportant de 6 à 24 atomes de carbone et le groupe aryle désignant de préférence un groupe phényle ou benzyle. On peut également utiliser les esters d'alkyle en C₆-C₂₄ et d'acides polyglycoside-carboxyliques tels que les glucoside-citrates d'alkyle, les polyglycoside-tartrates d'alkyle, et les polyglycoside-sulfosuccinates d'alkyle ; les alkylsulfosuccinamates, les acyliséthionates et les N-acyltaurates, le groupe alkyle ou acyle de tous ces composés comportant de 12 à 20 atomes de carbone. Parmi les tensioactifs anioniques encore utilisables, on peut également citer les acyllactylates dont le groupe acyle comporte de 8 à 20 atomes de carbone.

En outre, on peut encore citer les acides d'alkyl-D-galactoside uroniques et leurs sels ainsi que les acides alkyl(C6-C24)éther-carboxyliques polyoxyalkylénés, les acides alkyl(C₆-C₂₄)aryl(C₆-C₂₄)éther-carboxyliques polyoxyalkylénés, les acides alkyl(C₆-C₂₄)amidoéther carboxyliques polyoxyalkylénés et leurs sels, en particulier ceux comportant de 2 à 50 groupements oxyde d'éthylène, et leurs mélanges.

Les tensioactifs anioniques tels que décrits ci-dessus, peuvent être utilisés seuls ou en mélange. On utilise de préférence les alkylsulfates, les alkyléthersulfates et les alkyléthercarboxylates, et leurs mélanges, en particulier sous forme de leurs sels de métaux alcalins ou alcalino-terreux, d'ammonium, d'amine ou d'aminoalcool.

Les agents tensioactifs amphotères, convenant dans la présente invention, peuvent être notamment des dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique hydrosolubilisant tel que, par exemple, un groupe carboxylate, sulfonate, sulfate, phosphate ou phosphonate ; on peut citer encore les alkyl(C₈-C₂₀)bétaïnes, les sulfobétaïnes, les alkyl(C₈-C₂₀)amidoalkyl(C₆-C₈)-bétaïnes ou les alkyl(C₈-C₂₀)amidoalkyl(C₆-C₈)sulfobétaïnes ; et leurs mélanges.

Parmi les dérivés d'amines, on peut citer les produits commercialisés sous la dénomination MIRANOL®, tels que décrits dans les brevets US-2 528 378 et US-2 781 354 et classés dans le dictionnaire CTFA, 3ème édition, 1982, sous les dénominations Amphocarboxy-glycinate et Amphocarboxypropionate de structures respectives (1) et (2) :

Rₐ-CONHCH₂CH₂-N(R_{b})(R_{c})(CH₂COO-) (1)

dans laquelle :
Rₐ représente un groupe alkyle dérivé d'un acide Ra-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
   et

   Rₐ'-CONHCH₂CH₂-N(B)(C) (2)
dans laquelle :
B représente -CH₂CH₂OX',
C représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Ra' représente un groupe alkyle d'un acide Rₐ'-COOH présent dans l'huile de coprah ou dans l'huile de lin hydrolysée, un groupe alkyle, notamment en C₁₇ et sa forme iso, un groupe en C17 insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé sous la dénomination commerciale MIRANOL® C2M concentré par la société RHODIA.

Les tensioactifs **détergents,** peuvent être utilisés dans la composition de la présente invention en une quantité totale variant de 1 à 50 % en poids, de préférence de 5 à 35 % en poids et mieux encore, de 8 à 25 % en poids, par rapport au poids total de la composition.

Dans les compositions conformes à l'invention, on utilise de préférence des mélanges est un mélange constitué d'au moins un agent tensioactif anionique et d'au moins un agent tensioactif amphotère.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodium cocoamphodiacétate disodiumcocoamphodipropionate ou sodiumcocoamphopropionate commercialisés notamment par la société RHODIA sous la dénomination commerciale "MIRANOL^{®} C2M CONC" ou sous la dénomination MIRANOL^{®} C32;
- soit un agent tensioactif amphotère de type zwittérionique tel que les alkylbétaïnes en particulier la cocoylbétaïne commercialisée sous la dénomination "DEHYTON® AB 30" en solution aqueuse à 32 % de MA par la société HENKEL ou les alkylamidobétaïnes telles que la TEGOBETAINE^{®} F50 commercialisée par la société GLODSCHMIDT.

On utilisera plus particulièrement un agent tensioactif anionique choisi parmi les alkyl (C₁₂-C₁₄)éthersulfates de sodium oxyéthylénés à 2,2 moles d'oxyde d'éthylène comme le lauryl éther sulfate de sodium à 2,2 moles d'oxyde d'éthylène avec le cocoamphodiacétate de disodium commercialisé sous la dénomination commerciale MIRANOL® C2M concentré par la société RHODIA.

La composition de la présente invention conduit à des mousses présentant une rigidité élevée qui procure à l'utilisateur une sensation de densité lors de la prise en main. Les compositions moussantes conformes à l'invention produisent en général à la sortie du dispositif aérosol une mousse ayant une rigidité supérieure à 50 s et plus préférentiellement supérieure à 70 s, la rigidité étant mesurée selon le dispositif et la méthode suivants.

### Dispositif de mesure de la rigidité

Le dispositif de mesure décrit à la figure 1 comporte un cylindre vertical transparent en plexiglass de diamètre 50 mm, composé de trois parties principales: un socle 1 percé d'un trou de diamètre 24mm sur lequel est posée une valve 15, un corps inférieur cylindrique 2 de hauteur 104 mm vissé sur le socle, un corps supérieur cylindrique 3 de hauteur 95 mm vissé sur le corps inférieur 2 pour prolonger celui-ci axialement formant ainsi un logement cylindrique vertical ouvert à son extrémité supérieure.

Le dispositif de mesure comporte en outre un mobile coulissant dans le logement cylindrique. Il est composé d'une butée supérieure 12 en PVC de laquelle descendent deux suspentes de piston en inox 11. La butée 12 est constituée d'un disque de diamètre supérieur à celui du logement cylindrique. Les suspentes sont rattachées à la butée par deux vis 13 et sont enfoncées à leur extrémité inférieure dans un piston 4 en PVC de poids 119,3 g et de diamètre 45 mm. Dans ce piston est disposé un lest en inox 8, de poids 225 g et de longueur 58,6 mm. Le piston comporte des ailettes de guidage en PVC 7 régulièrement répartis à sa périphérie et s'étendant radialement. Les ailettes sont adaptées pour le guidage du piston suivant l'axe du logement cylindrique.

### méthode de mesure de la rigidité à partir du dispositif de mesure décrit ci dessus

Un dispositif aérosol à tester est placé une heure et demi dans un bain d'eau à 20+-1°C. Après avoir agité ce dispositif aérosol en faisant cinq allers et retours, celui ci est vidangé à 5 % pour obtenir un taux de remplissage du dispositif aérosol de 95 %. La valve du dispositif aérosol à tester est alors enclenchée sur la valve placée dans le socle du corps inférieur du dispositif de mesure afin de remplir le logement cylindrique de mousse. Cette opération doit être effectuée sans inclure d'air. On arase ensuite la surface supérieure du logement cylindrique. Le corps supérieur est alors vissé au corps inférieur. Le mobile qui comporte le lest est introduit dans le logement cylindrique depuis l'extrémité ouverte. Quand le fond du mobile est en contact avec la mousse, on déclenche le chronomètre. Le chronomètre est arrêté lorsque le mobile a terminé sa descente, c'est-à-dire quand la butée 12 entre en contact avec le sommet du corps supérieur.

La rigidité est mesurée par le temps en seconde que met le mobile pour descendre dans le logement cylindrique.

Le milieu aqueux cosmétiquement acceptable de la composition conforme à l'invention est généralement constitué par de l'eau ou par un mélange d'eau et d'au moins un solvant organique. A titre de solvant organique, on peut utiliser les polyols comme le propylène glycol, la glycérine, l'hexylène glycol, l'isoprène glycol, le néopentyl glycol, les polyéthylène glycols ou les éthers de polyols. On préfère plus particulièrement utiliser un mélange de glycérine et d'hexylène glycol. Les solvants peuvent être présents dans des proportions de préférence comprises entre 0 et 20 % en poids environ par rapport au poids total de la composition, et encore plus préférentiellement entre 1 et 10% en poids environ.

La composition conforme à l'invention comprend de préférence en outre au moins un adjuvant choisi parmi les agents opacifiants, les agents épaississants, les agents conditionneurs tels que les huiles végétales ou minérales, les cires, les céramides ou les polymères cationiques autres que les polymères de vinylpyrrolidone, les polymères amphotères ou les silicones ; les humectants tel que le sorbitol, les émollients, les agents conservateurs, les filtres UV, les agents de pH, les électrolytes tels que le chlorure de sodium, les agents séquestrants, les colorants, les parfums et les peptisants de parfums.

Le pH de la composition conforme à l'invention est généralement ajusté de 4 à 10 au moyen d'agents acidifiants ou alcalinisants bien connus de la technique en cosmétique.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante : 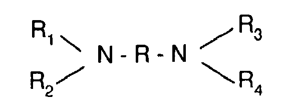 dans laquelle R est un reste propylène éventuellement substitué par un groupement hydroxyle ou un radical alkyle en C₁-C₄ ; R1, R₂, R₃ et R₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides organiques ou minéraux dont on préfère plus particulièrement l'acide citrique, l'acide tartrique, ou l'acide lactique.

Parmi les agents opacifiants utilisables dans la composition conforme à l'invention, on peut citer par exemple les alcools gras à chaîne longue, les esters gras de polyol et les diéthers gras comme par exemple le distéaryl éther, bien connus de l'homme de l'art. On préfère notamment utiliser selon l'invention, le mélange aqueux de copolymère styrène/acrylate de sodium, lauryl sulfate de sodium, sorbate de potassium, polyéthylène glycol (7 OE) éther d'alcool tridécylique (appellation CTFA Trideceth-7) vendu sous la dénomination OPACIFIER 680^{®} par la société ROHM & HAAS.

Parmi les agents épaississants, on peut citer notamment les acides polyacryliques réticulés vendus sous la dénomination CARBOPOL^{®}, et les polymères épaississants associatifs anioniques, non ioniques, cationiques ou amphotères comportant au moins une chaîne grasse; on peut également citer le chlorure de sodium que l'on préfère plus particulièrement utiliser dans la proportion de 1 à 2% en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Ces additifs sont présents dans la composition selon l'invention en une quantité allant de 0 à 20 % en poids par rapport au poids total de la composition.

Un autre objet de l'invention consiste en l'utilisation de ladite composition pour la préparation d'un produit cosmétique ou dermatologique et notamment d'un produit de nettoyage des matières kératiniques.

Les compositions conformes à la présente invention peuvent être utilisées comme produits de douche, comme produits pour le bain moussant, comme produits de nettoyage du visage (démaquillants), des produits pour le rasage. Des applications capiiiaires peuvent être prévues comme les shampooings et les après-shampooings.

Un autre objet de l'invention consiste en un dispositif aérosol comprenant dans un même compartiment la composition moussante selon la présente invention. Selon un mode de réalisation particulier, le dispositif aérosol de la présente invention est un dispositif à un seul compartiment dans lequel la composition moussante de l'invention est conditionnée.

Un autre objet de l'invention consiste en un procédé de nettoyage des matières kératiniques consistant à produire une mousse à la sortie du dispositif aérosol dans lequel est conditionnée la composition de l'invention et à l'appliquer sur lesdits matières kératiniques puis après un éventuel temps de pose, à procéder au rinçage.

### EXEMPLE 1 : Invention

Dans cet exemple, on utilise un dispositif aérosol comprenant dans un même compartiment la composition suivante :

| | |
|---|---|
| Polyquaternium-11 | 0,28 g |
| Hydroxypropylméthylcellulose | 0,19 g |
| Lauryléther sulfate à 2 moles d'oxyde d'éthylène | 10,52 g |

| | |
|---|---|
| Disodium cocoamphodiacétate | 2,14 g |
| Polyols émollients, hydratants, solvants | 3,85 g |
| Mélange de propulseurs hydrocarbonés | 6 g |
| Sequestrant, Electrolyte, Conservateurs, Parfum, Peptisant | qs |
| Eau | qsp 100 g |

La rigidité de la mousse à la sortie du dispositif aérosol est mesurée selon le dispositif de mesure et la méthode décrite précédemment. Les résultats sont reportés dans le tableau 1 ci dessous.

### EXEMPLE 2: comparatif

Dans cet exemple, on utilise un dispositif aérosol comprenant dans un même compartiment la composition suivante :

| | |
|---|---|
| Polyquaternium-11 | 0,28 g |
| Lauryléther sulfate à 2 moles d'oxyde d'éthylène | 10,52 g |
| Disodium cocoamphodiacétate | 2,14 g |
| Polyols émollients, hydratants, solvants | 3,85 g |
| Mélange de propulseurs hydrocarbonés | 6 g |
| Sequestrant, Electrolyte, Conservateurs, Parfum, Peptisant | qs |
| Eau | qsp 100 g |

La rigidité de la mousse à la sortie du dispositif aérosol est mesurée selon le dispositif de mesure et la méthode décrite précédemment. Les résultats sont reportés dans le tableau 1 ci dessous.

### EXEMPLE 3 : comparatif

Dans cet exemple, on utilise un dispositif aérosol comprenant dans un même compartiment la composition suivante :

| | |
|---|---|
| Hydroxypropylmethylcellulose | 0,19 g |
| Lauryléther sulfate à 2 moles d'oxyde d'éthylène | 10,52 g |

| | |
|---|---|
| Disodium cocoamphodiacétate | 2,14 g |
| Polyols émollients, hydratants, solvants | 3,85 g |
| Mélange de propulseurs hydrocarbonés | 6 g |
| Sequestrant, Electrolyte, Conservateurs, Parfum, Peptisant | qs |
| Eau | qsp 100 g |

La rigidité de la mousse à la sortie du dispositif aérosol est mesurée selon le dispositif de mesure et la méthode décrite précédemment. Les résultats sont reportés dans le tableau 1 ci dessous.

### EXEMPLE 4 : Témoin :

Dans cet exemple, on utilise un dispositif aérosol comprenant dans un même compartiment la composition suivante :

| | |
|---|---|
| Lauryléther sulfate à 2 moles d'oxyde d'éthylène | 10,52 g |
| Disodium cocoamphodiacétate | 2,14 g |
| Polyols émollients, hydratants, solvants | 3,85 g |
| Mélange de propulseurs hydrocarbonés | 6 g |
| Sequestrant, Electrolyte, Conservateurs, Parfum, Peptisant | qs |
| Eau | qsp 100 g |

La rigidité de la mousse à la sortie du dispositif aérosol est mesurée selon le dispositif de mesure et la méthode décrite précédemment. Les résultats sont reportés dans le tableau 1 ci dessous.

**TABLEAU 1**

| **Composition** | **Rigidité (en secondes)** |
|---|---|
| Exemple 1 (invention) | 101 |
| Exemple 2 | 45 |
| Exemple 3 | 67 |
| Exemple 4 | 25 |

La composition aérosol selon l'invention produit une mousse dont la rigidité est 1,5 à 4 fois plus élevée que celle produite par les compositions des exemples 2, 3 ou 4.

## Revendications

1. Composition moussante comprenant :
(a) au moins un agent propulseur et
(b) une phase liquide contenant dans un milieu aqueux cosmétiquement acceptable :
(i) un mélange de tensio-actifs détergents constitué d'au moins un agent tensioactif anionique et d'au moins un agent tensioactif amphotère;
(ii) au moins un polymère cationique de vinylpyrrolidone choisi parmi les copolymères vinylpyrrolidone / méthacrylate de diméthylaminoéthyle quaternisés par du sulfate de diéthyle et
(iii) au moins un éther de cellulose non ionique choisi parmi les alkylcelluloses ; les hydroxyalkylcelluloses ; les celluloses mixtes hydroxyalkyl-alkylcelluloses.

2. Composition selon la revendication 1 où le polymère cationique de vinylpyrrolidone est le polyquaternium-11.

3. Composition selon l'une quelconque des revendications 1 et à 2 3 où la concentration en polymère cationique de vinylpyrrolidone varie de 0,01 à 5 % en poids par rapport au poids total de la composition.

4. Composition selon la revendication 3, où la concentration en polymère de vinylpyrrolidone varie de 0,05 à 2 % en poids par rapport au poids total de la composition.

5. composition selon l'une quelconque des revendications 1 à 4 où le composé cellulosique est une hydroxypropyl-méthylcellulose.

6. composition selon l'une quelconque des revendications 1 à 5 où le ou les composés cellulosiques représentent de 0,01 à 5 % en poids par rapport au poids total de la composition.

7. Composition selon la revendication 6 où le ou les composés cellulosiques représentent de 0,05 à 2 % en poids par rapport au poids total de la composition.

8. Composition selon l'une quelconque des revendications 1 à 7 où l'agent propulseur est choisi parmi les gaz hydrocarbonés, les gaz fluorés, les gaz hydrofluorocarbonés, le dimethyl ether, l'azote, l'air et le dioxyde de carbone et leurs mélanges.

9. Composition selon la revendication 8 où l'agent propulseur est choisi parmi les gaz hydrocarbonés ayant de 2 à 6 atomes de carbone.

10. Composition selon la revendication 9 où l'agent propulseur est un mélange iso-butane, propane, n-butane.

11. Composition selon l'une quelconque des revendications 1 à 10 où l'agent propulseur est présent à raison de 0,1 à 15% en poids par rapport au poids total de la composition.

12. Composition selon la revendication 13 où l'agent propulseur est présent à raison de 1 à 8% en poids par rapport au poids total de la composition.

13. Composition selon l'une quelconque des revendications 1 à 12 revendication 22, où le mélange de tensio-actifs est constitué :
- d'au moins un agent tensioactif anionique choisi parmi les alkyl(C₁₂-C₁₄) sulfates de sodium, de triéthanolamine ou d'ammonium, les alkyl (C₁₂-C₁₄)éthersulfates de sodium oxyéthylénés à 2,2 moles d'oxyde d'éthylène, le cocoyl iséthionate de sodium et l'alphaoléfine(C₁₄-C₁₆) sulfonate de sodium et leurs mélange et :
- d'au moins un agent tensioactif amphotère choisis parmi :
(1) les dérivés d'amine dénommés disodium cocoamphodiacétate disodiumcocoamphodipropionate ou sodiumcocoamphopropionate ;
(2) les alkylbétaïnes ou les alkylamidobétaïnes.

14. Composition selon la revendication 13, où le mélange de tensio-actifs est constitué :
- d'au moins un agent tensioactif anionique choisi parmi les alkyl (C₁₂-C₁₄)éthersulfates de sodium oxyéthylénés à 2,2 moles d'oxyde d'éthylène et
- et du cocoamphodiacétate de disodium.

15. Composition selon l'une quelconque des revendications 1 à 14 où le ou les tensioactifs détergents sont présents en une quantité totale variant de 1 à 50 % en poids, par rapport au poids total de la composition.

16. Composition selon la revendication 15 où le ou les tensioactifs détergents sont présents en une quantité totale variant 5 à 35 % en poids par rapport au poids total de la composition.

17. Composition selon la revendication 16 où le ou les tensioactifs détergents sont présents en une quantité totale variant de 8 à 25 % en poids, par rapport au poids total de la composition.

18. Composition selon l'une quelconque des revendications 1 à 17, **caractérisée par le fait qu'**elle produit en général à la sortie du dispositif aérosol une mousse ayant une rigidité supérieure à 50 s

19. Composition selon la revendication 18, **caractérisée par le fait qu'**elle produit à la sortie du dispositif aérosol une mousse ayant une rigidité supérieure à 70 s.

20. Composition selon l'une quelconque des revendications 1 à 19 où milieu aqueux cosmétiquement acceptable est constitué par de l'eau ou par un mélange d'eau et d'au moins un solvant organique.

21. Composition selon la revendication 20 où le ou les solvants organiques sont présents dans des proportions allant de 0 à 20 % en poids par rapport au poids total de la composition, et de préférence allant de 1 à 10% en poids.

22. Composition selon la revendication 20 ou 21, où le ou les solvants organiques sont choisis parmi les polyols, les polyéthylène glycols ou les éthers de polyols.

23. Composition selon la revendication 22 33, où les solvants organiques sont un mélange de glycérine et d'hexylène glycol.

24. Composition selon l'une quelconque des revendications 1 à 23 34, **caractérisée par le fait qu'**elle comprend en outre au moins un adjuvant choisi parmi les agents opacifiants, les agents épaississants, les agents conditionneurs ; les humectants, les émollients, les agents conservateurs, les filtres UV, les agents de pH, les électrolytes, les agents séquestrants, les colorants, les parfums et les peptisants de parfums.

25. Composition selon l'une quelconque des revendications 1 à 32 **caractérisée par le fait que** le pH varie de 4 à 10.

26. Utilisation d'une composition selon l'une quelconque des revendications 1 à 25 pour la préparation d'un produit cosmétique ou dermatologique.

27. Utilisation selon la revendication 26 où ledit produit cosmétique ou dermatologique est un produit de nettoyage des matières kératiniques.

28. Utilisation selon la revendication 26 où ledit produit cosmétique ou dermatologique est un produit pour la douche ; un bain moussant; un démaquillant; un produit pour le rasage; un shampooing ou un après shampooing.

29. Procédé de nettoyage des matières kératiniques consistant à produire une mousse à la sortie d'un dispositif aérosol dans lequel est conditionnée une composition selon l'une quelconque des revendications 1 à 25 36 et à l'appliquer sur lesdits matières kératiniques puis après un éventuel temps de pose, à procéder au rinçage.

30. Dispositif aérosol comprenant dans un même compartiment la composition moussante telle que définie selon l'une quelconque des revendications 1 à 25 36.

31. Dispositif selon la revendication 30 comprenant un seul compartiment dans lequel la composition moussante est conditionnée.

## Claims

1. Foaming composition comprising:
(a) at least one propellant, and
(b) a liquid phase containing, in a cosmetically acceptable medium:
(i) a mixture of detergent surfactants consisting of at least one anionic surfactant and of at least one amphoteric surfactant;
(ii) at least one cationic vinylpyrrolidone polymer chosen from vinylpyrrolidone/dimethylaminoethyl methacrylate copolymers quaternized with diethyl sulphate, and
(iii) at least one non-ionic cellulose ether chosen from alkylcelluloses; hydroxyalkylcelluloses; and hydroxyalkyl-alkylcellulose mixed celluloses.

2. Composition according to Claim 1, where the cationic vinylpyrrolidone polymer is polyquaternium-11.

3. Composition according to either one of Claims 1 and 2, where the concentration of cationic vinylpyrrolidone polymer ranges from 0.01% to 5% by weight relative to the total weight of the composition.

4. Composition according to Claim 3, where the concentration of vinylpyrrolidone polymer ranges from 0.05% to 2% by weight relative to the total weight of the composition.

5. Composition according to any one of Claims 1 to 4,
where the cellulosic compound is a hydroxypropylmethylcellulose.

6. Composition according to any one of Claims 1 to 5,
where the cellulosic compound(s) represent(s) from 0.01% to 5% by weight relative to the total weight of the composition.

7. Composition according to Claim 6, where the cellulosic compounds represent from 0.05% to 2% by weight relative to the total weight of the composition.

8. Composition according to any one of Claims 1 to 7,
where the propellant is chosen from hydrocarbon-based gases, fluorinated gases, hydrofluorocarbon-based gases, dimethyl ether, nitrogen, air and carbon dioxide, and mixtures thereof.

9. Composition according to Claim 8, where the propellant is chosen from hydrocarbon-based gases containing from 2 to 6 carbon atoms.

10. Composition according to Claim 9, where the propellant is a mixture of isobutane, propane and n-butane.

11. Composition according to any one of Claims 1 to 10, where the propellant is present in a proportion of from 0.1% to 15% by weight relative to the total weight of the composition.

12. Composition according to Claim 11, where the propellant is present in a proportion of from 1% to 8% by weight relative to the total weight of the composition.

13. Composition according to any one of Claims 1 to 12, where the mixture of surfactants consists:
- of at least one anionic surfactant chosen from sodium (C₁₂-C₁₄)alkyl sulphates, triethanolamine (C₁₂-C₁₄)alkyl sulphates, ammonium (C₁₂-C₁₉)alkyl sulphates, oxyethylenated sodium (C₁₂-C₁₄)alkyl ether sulphates comprising 2.2 mol of ethylene oxide, sodium cocoyl isethionate and sodium alpha-(C₁₄-C₁₈)olefinsulphonate, and mixtures thereof, and
- of at least one amphoteric surfactant chosen from:
(1) the amine derivatives known as disodium cocoamphodiacetate, disodium cocoamphodipropionate or sodium cocoamphopropionate;
(2) alkylbetaines or alkylaminobetaines.

14. Composition according to Claim 13, where the mixture of surfactants consists:
- of at least one anionic surfactant chosen from oxyethylenated sodium (C₁₂-C₁₄)alkyl ether sulphates comprising 2.2 mol of ethylene oxide, and
- of disodium cocoamphodiacetate.

15. Composition according to any one of Claims 1 to 14, where the detergent surfactant(s) is (are) present in a total amount ranging from 1% to 50% by weight relative to the total weight of the composition.

16. Composition according to Claim 15, where the detergent surfactant(s) is (are) present in a total amount ranging from 5% to 35% by weight relative to the total weight of the composition.

17. Composition according to Claim 16, where the detergent surfactant(s) is (are) present in a total amount ranging from 8% to 25% by weight relative to the total weight of the composition.

18. Composition according to any one of Claims 1 to 17, **characterized in that** it produces, in general, at the outlet of the aerosol device a foam having a rigidity of greater than 50 s.

19. Composition according to Claim 18, **characterized in that** it produces at the outlet of the aerosol device a foam having a rigidity of greater than 70 s.

20. Composition according to any one of Claims 1 to 19, where the cosmetically acceptable aqueous medium consists of water or of a mixture of water and at least one organic solvent.

21. Composition according to Claim 20, where the organic solvent(s) is (are) present in proportions ranging from 0% to 20% by weight relative to the total weight of the composition, and preferably ranging from 1% to 10% by weight.

22. Composition according to Claim 20 or 21, where the organic solvent(s) is (are) chosen from polyols, polyethylene glycols or polyol ethers.

23. Composition according to Claim 22, where the organic solvents are a mixture of glycerol and hexylene glycol.

24. Composition according to any one of Claims 1 to 23, **characterized in that** it also comprises at least one adjuvant chosen from opacifiers, thickeners, conditioners; humectants, emollients, preserving agents, UV-screening agents, pH agents, electrolytes, sequestering agents, dyes, fragrances and fragrance peptizers.

25. Composition according to any one of Claims 1 to 24, **characterized in that** the pH ranges from 4 to 10.

26. Use of a composition according to any one of Claims 1 to 25, for the preparation of a cosmetic or dermatological product.

27. Use according to Claim 26, where said cosmetic or dermatological product is a product for cleansing keratin materials.

28. Use according to Claim 26, where said cosmetic or dermatological product is a shower product; a bubble bath; a makeup-removing product; a shaving product; a shampoo or a conditioner.

29. Method for cleansing keratin materials, consisting in producing a foam at the outlet of an aerosol device in which a composition according to any one of Claims 1 to 25 is packaged, and in applying it to said keratin materials and then, after an optional leave-on time, in rinsing it off.

30. Aerosol device comprising, in the same compartment, the foaming composition as defined in any one of Claims 1 to 25.

31. Device according to Claim 30, comprising a single compartment in which the foaming composition is packaged.

## Patentansprüche

1. Schaumbildende Zusammensetzung, die enthält:
(a) mindestens ein Treibmittel und
(b) eine flüssige Phase, die in einem kosmetisch akzeptablen, wässrigen Medium enthält:
(i) ein Gemisch aus reinigenden grenzflächenaktiven Stoffen, das aus mindestens einem anionischen grenzflächenaktiven Stoff und mindestens einem amphoteren grenzflächenaktiven Stoff besteht;
(ii) mindestens ein kationisches Vinylpyrrolidonpolymer, das unter den Vinylpyrrolidon/Dimethylaminoethylmethacrylat-copolymeren, die mit Diethylsulfat quaternisiert sind, ausgewählt ist, und
(iii) mindestens einen nichtionischen Celluloseether, der unter den Alkylcellulosen; Hydroxyalkylcellulosen; gemischten Hydroxyalkyl-alkylcellulosen ausgewählt ist.

2. Zusammensetzung nach Anspruch 1, wobei das kationische Vinylpyrrolidonpolymer das Polyquaternium-11 ist.

3. Zusammensetzung nach einem der Ansprüche 1, und 2, wobei die Konzentration des kationischen Vinylpyrrolidonpolymers im Bereich von 0,01 bis 5 Gew.-%, bezogen auf das Gesamtgewicht der Zusammensetzung, liegt.

4. Zusammensetzung nach Anspruch 3, wobei die Konzentration des Vinylpyrrolidonpolymers im Bereich von 0,05 bis 2 Gew.-%, bezogen auf das Gesamtgewicht der Zusammensetzung, liegt.

5. Zusammensetzung nach einem der Ansprüche 1 bis 4, wobei die Celluloseverbindung eine Hydroxypropyl-methylcellulose ist.

6. Zusammensetzung nach einem der Ansprüche 1 bis 5, wobei die Celluloseverbindung(en) 0,01 bis 5 Gew.-%, bezogen auf das Gesamtgewicht der Zusammensetzung, ausmachen.

7. Zusammensetzung nach Anspruch 6, wobei die Celluloseverbindung(en) 0,05 bis 2 Gew.-%, bezogen auf das Gesamtgewicht der Zusammensetzung, ausmachen.

8. Zusammensetzung nach einem der Ansprüche 1 bis 7, wobei das Treibmittel unter den Kohlenwasserstoffgasen, fluorierten Gasen, Fluorkohlenwasserstoffgasen, Dimethylether, Stickstoff, Luft und Kohlendioxid und deren Gemischen ausgewählt ist.

9. Zusammensetzung nach Anspruch 8, wobei das Treibmittel unter den Kohlenwasserstoffgasen mit 2 bis 6 Kohlenstoffatomen ausgewählt ist.

10. Zusammensetzung nach Anspruch 9, wobei das Treibmittel ein Gemisch aus Isobutan, Propan und *n*-Butan ist.

11. Zusammensetzung nach einem der Ansprüche 1 bis 10, wobei das Treibmittel in einer Menge von 0,1 bis 15 Gew.-%, bezogen auf das Gesamtgewicht der Zusammensetzung, enthalten ist.

12. Zusammensetzung nach Anspruch 13, wobei das Triebmittel in einer Menge von 1 bis 8 Gew.-%, bezogen auf das Gesamtgewicht der Zusammensetzung, enthalten ist.

13. Zusammensetzung nach einem der Ansprüche 1 bis 12, wobei das Gemisch aus grenzflächenaktiven Stoffen besteht aus:
- mindestens einem anionischen grenzflächenaktiven Stoff, der unter den Natriuxnalkyl(C₁₂-C₁₄)sulfaten, Triethanolalkyl(C₁₂₋₁₄)-sulfaten oder Ammoniumalkyl(C₁₂₋₁₄)sulfaten, Natriumalkyl-(C₁₂₋₁₄)ethersulfaten, die mit 2,2 mol Ethylenoxid ethoxyliert sind, Natriumcocoylisethionat und Natriumalphaolefin(C₁₄₋₁₆)-sulfonat und deren Gemischen ausgewählt ist, und
- mindestens einem amphoteren grenzflächenaktiven Stoff, der ausgewählt ist unter:
(1) Aminderivaten, die als Disodium Cocoamphodiacetate, Disodium Cocoamphodipropionate oder Sodium Cocoamphopropionate bezeichnet werden;
(2) Alkylbetainen oder Alkylamidobetaninen.

14. Zusammensetzung nach Anspruch 13, wobei das Gemisch aus grenzflächenaktiven Stoffen besteht aus:
- mindestens einem anionischen grenzflächenaktiven Stoff, der unter den mit 2,2 mol Ethylenoxid ethoxylierten Natriumalkyl(C₁₂₋₁₄)ethersulfaten und
- Dinatriumcocoamphodiacetat ausgewählt ist.

15. Zusammensetzung nach einem der Ansprüche 1 bis 14, wobei der oder die reinigenden grenzflächenaktiven Stoffe in einer Gesamtmenge von 1 bis 50 Gew.-%, bezogen auf das Gesamtgewicht der Zusammensetzung, enthalten sind.

16. Zusammensetzung nach Anspruch 15, wobei der oder die reinigenden grenzflächenaktiven Stoffe in einer Gesamtmenge von 5 bis 35 Gew.-%, bezogen auf das Gesamtgewicht der Zusammensetzung, enthalten sind.

17. Zusammensetzung nach Anspruch 16, wobei der oder die reinigenden grenzflächenaktiven Stoffe in einer Gesamtmenge von 8 bis 25 Gew.-%, bezogen auf das Gesamtgewicht der Zusammensetzung, enthalten sind.

18. Zusammensetzung nach einem der Ansprüche 1 bis 17, **dadurch gekennzeichnet, dass** sie am Auslass der Aerosolvorrichtung einen Schaum mit einer Steifigkeit über 50 s bildet.

19. Zusammensetzung nach Anspruch 18, **dadurch gekennzeichnet, dass** sie am Auslass der Aerosolvorrichtung einen Schaum mit einer Steifigkeit über 70 s bildet.

20. Zusammensetzung nach einem der Ansprüche 1 bis 19, wobei das kosmetisch akzeptable, wässrige Medium aus Wasser oder einem Gemisch von Wasser und mindestens einem organischen Lösungsmittel besteht.

21. Zusammensetzung nach Anspruch 20, wobei das oder die organischen Lösungsmittel in Mengenanteilen von 0 bis 20 Gew.-%, bezogen auf das Gesamtgewicht der Zusammensetzung, und vorzugsweise 1, bis 10 Gew.-% enthalten sind.

22. Zusammensetzung nach Anspruch 20 oder 21, wobei das oder die organischen Lösungsmittel unter den Polyolen, Polyethylenglycolen oder Polyolethern ausgewählt sind.

23. Zusammensetzung nach Anspruch 22, wobei es sich bei den organischen Lösungsmitteln um ein Gemisch aus Glycerin und Hexylenglycol handelt.

24. Zusammensetzung nach einem der Ansprüche 1 bis 23, **dadurch gekennzeichnet, dass** sie ferner mindestens einen Zusatzstoff enthält, der unter den Trübungsmitteln, Verdickungsmitteln, Konditioniermitteln; Feuchthaltemitteln, Emollientien, Konservierungsmitteln, UV-Filtern, pH-Reglern, Elektrolyten, Maskierungsmitteln, Farbmitteln, Parfums und Peptisierungsmitteln für das Parfum ausgewählt ist.

25. Zusammensetzung nach einem der Ansprüche 1 bis 32, **dadurch gekennzeichnet, dass** der pH-Wert im Bereich von 4 bis 10 liegt.

26. Verwendung einer Zusammensetzung nach einem der Ansprüche 1 bis 25 zur Herstellung eines kosmetischen oder dermatologischen Produkts.

27. Verwendung nach Anspruch 26, wobei das kosmetische oder dermatologische Produkt ein Produkt für die Reinigung von Keratinsubstanzen ist.

28. Verwendung nach Anspruch 26, wobei das kosmetische oder dermatologische Produkt ein Produkt für die Dusche, ein Schaumbad; ein Abschminkmittel; ein Produkt für die Rasur; ein Haarwaschmittel oder ein nach der Haarwäsche anzuwendendes Produkt ist.

29. Verfahren zur Reinigung von Keratinsubstanzen, das darin besteht, am Auslass einer Aerosolvorrichtung, in der eine Zusammensetzung nach einem der Ansprüche 1 bis 25 konfektioniert ist, einen Schaum zu bilden und diesen auf die Keratinsubstanzen aufzubringen und anschließend nach einer gegebenenfalls abgewarteten Einwirkzeit zu spülen.

30. Aerosolvorrichtung, die in einer Abteilung die schaumbildende Zusammensetzung nach einem der Ansprüche 1 bis 25 enthält.

31. Vorrichtung nach Anspruch 30, die nur eine Abteilung aufweist, in der die schaumbildende Zusammensetzung konfektioniert ist.
